# EUROPÄISCHE PATENTSCHRIFT

(11) **EP 2 912 583 B1**
(45) Veröffentlichungstag und Bekanntmachung des Hinweises auf die Patenterteilung: **17.03.2021**
(21) Anmeldenummer: 13782969.3
(22) Anmeldetag: 17.10.2013
(51) Int. Cl.: G16H 40/63, A61M 1/14

(54) **VORRICHTUNG, SYSTEM UND VERFAHREN ZUR ÜBERWACHUNG, INFORMATIONSANZEIGE UND BEDIENUNG VON MEDIZINISCHEN FLUIDMANAGEMENTGERÄTEN**
APPARATUS, SYSTEM AND MONITORING METHOD, INFORMATION DISPLAY AND CONTROL OF MEDICAL FLUID MANAGEMENT APPLIANCES
DISPOSITIF, SYSTÈME ET PROCÉDÉ DE SURVEILLANCE, D'AFFICHAGE D'INFORMATIONS ET DE COMMANDE D'APPAREILS DE GESTION DE FLUIDES MÉDICAUX

(30) Priorität: 25.10.2012 DE 102012020945; 25.10.2012 US 201261718412 P
(43) Veröffentlichungstag der Anmeldung: 02.09.2015
(73) Patentinhaber: Fresenius Medical Care Deutschland GmbH, 61352 Bad Homburg (DE)
(72) Erfinder: MÜLLER, Carsten, 97502 Euerbach (DE)
(74) Vertreter: Dreyhsig, Jörg
(86) Internationale Anmeldenummer: PCT/EP2013/003124
(87) Internationale Veröffentlichungsnummer: WO 2014/063798

(56) Entgegenhaltungen:
- WO-A1-01/28416
- WO-A1-2007/126360
- WO-A2-2009/122277
- US-A1- 2010 137 693
- US-A1- 2011 054 352
- US-A1- 2011 054 378
- US-A1- 2012 252 543
- KIRSCH C ET AL: "MONITORING CHRONICALLY ILL PATIENTS USING MOBILE TECHNOLOGIES", IBM SYSTEMS JOURNAL, IBM CORP. ARMONK, NEW YORK, US, Bd. 46, Nr. 1, 1. Januar 2007 (2007-01-01) , Seiten 85-93, XP001506014, ISSN: 0018-8670

## Beschreibung

Die Erfindung betrifft das Gebiet von medizinischen Fluidmanagementgeräten, insbesondere eine Vorrichtung, ein System und ein Verfahren zur Überwachung, Informationsanzeige und Bedienung eines medizinischen Fluidmanagementgerätes.

### Stand der Technik

Unter medizinischen Fluidmanagementgeräten werden hierbei insbesondere Geräte zur Leitung, Behandlung und/oder Verteilung von Flüssigkeiten und/oder Gasen verstanden, bei denen über eine Fluidleitung Fluid zwischen einem Patienten und einer Fluidbehandlungskomponente und/oder einer Fluidquelle transportiert werden.

Unter Fluidmanagementgeräten werden insbesondere auch Fluidbehandlungsgeräte wie Blutbehandlungsgeräte verstanden, bei denen ein Fluid eines Patienten über eine Fluidleitung einer Fluidbehandlungskomponente zugeführt, durch die Fluidbehandlungskomponente behandelt und über die Fluidleitung, die in einen arteriellen und einen venösen Zweig aufgeteilt werden kann, wieder an den Patienten zurückgegeben wird. Beispiele für solche Blutbehandlungsgeräte sind insbesondere Hämodialysegeräte. Ein solches Blutbehandlungsgerät ist Gegenstand der DE 198 49 787 C1 der Anmelderin.

Die Dialyse ist ein Verfahren zur Blutreinigung von Patienten mit akuter oder chronischer Niereninsuffizienz. Grundsätzlich unterscheidet man hierbei Verfahren mit einem extrakorporalen Blutkreislauf, wie die Hämodialyse, die Hämofiltration oder die Hämodiafiltration und der Peritonealdialyse, die keinen extrakorporalen Blutkreislauf aufweist.

Das Blut wird bei der Hämodialyse in einem extrakorporalen Kreislauf durch die Blutkammer eines Dialysators geleitet, die über eine semipermeable Membran von einer Dialysierflüssigkeitskammer getrennt ist. Die Dialysierflüssigkeitskammer wird von einer die Blutelektrolyte in einer bestimmten Konzentration enthaltenen Dialysierflüssigkeit durchströmt. Die Stoffkonzentration der Blutelektrolyte in der Dialysierflüssigkeit entspricht dabei der Konzentration im Blut eines Gesunden. Während der Behandlung werden das Blut des Patienten und die Dialysierflüssigkeit an beiden Seiten der semipermeablen Membran im Allgemeinen im Gegenstrom mit einer vorgegebenen Flussrate vorbeigeführt. Die harnpflichtigen Stoffe diffundieren durch die Membran von der Blutkammer in die Kammer für Dialysierflüssigkeit, während gleichzeitig im Blut und in der Dialysierflüssigkeit vorhandene Elektrolyte von der Kammer höherer Konzentration zur Kammer niedrigerer Konzentration diffundieren. Wird an der Dialysemembran ein Druckgradient von der Blutseite zur Dialysatseite aufgebaut, beispielsweise durch eine Pumpe, die flussabwärts des Dialysefilters auf der Dialysatseite Dialysat aus dem Dialysatkreislauf entzieht, tritt Wasser aus dem Patientenblut über die Dialysemembran in den Dialysatkreislauf über. Dieser Vorgang der Ultrafiltration führt zu einer gewünschten Entwässerung des Patientenbluts.

Bei der Hämofiltration wird dem Patientenblut durch Anlegen eines Transmembrandrucks im Dialysator Ultrafiltrat entzogen, ohne dass Dialysierflüssigkeit auf der dem Patientenblut gegenüber liegenden Seite der Membran des Dialysators vorbeigeführt wird. Zusätzlich kann dem Patientenblut eine sterile und pyrogenfreie Substituatslösung zugesetzt werden. Je nachdem, ob diese Substituatslösung stromaufwärts des Dialysators zugesetzt wird oder stromabwärts, spricht man von Prä- oder Postdilution. Der Stoffaustausch erfolgt bei der Hämofiltration konvektiv.

Die Hämodiafiltration kombiniert die Verfahren der Hämodialyse und der Hämofiltration. Es findet sowohl ein diffusiver Stoffaustausch zwischen Patientenblut und Dialysierflüssigkeit über die semipermeable Membran eines Dialysators statt, als auch eine Abfiltrierung von Plasmawasser durch einen Druckgradienten an der Membran des Dialysators.

Die Verfahren der Hämodialyse, der Hämofiltration und der Hämodiafiltration, im Folgenden unter dem Begriff Hämodialyse zusammengefasst, werden in der Regel mit automatischen Hämodialysegeräten durchgeführt, wie sie beispielsweise von der Anmelderin unter der Bezeichnung 5008 vertrieben werden.

Die Plasmapherese ist ein Blutbehandlungsverfahren, bei dem das Patientenblut in das Blutplasma und seine korpuskularen Bestandteile (Zellen) aufgetrennt wird. Das abgetrennte Blutplasma wird gereinigt oder durch eine Substitutionslösung ersetzt und das gereinigte Blutplasma oder die Substitutionslösung dem Patienten zurückgegeben.

Bei der Peritonealdialyse wird die Bauchhöhle eines Patienten über einen durch die Bauchdecke geführten Katheter mit einer Dialyseflüssigkeit befüllt, die ein Konzentrationsgefälle gegenüber den körpereigenen Flüssigkeiten aufweist. Über das als Membran wirkende Bauchfell (Peritoneum) treten die im Körper vorliegenden Giftstoffe in die Bauchhöhle über. Nach einigen Stunden wird die sich in der Bauchhöhle des Patienten befindliche, nunmehr verbrauchte Dialyseflüssigkeit ausgetauscht. Durch osmotische Vorgänge kann Wasser aus dem Blut des Patienten über das Bauchfell in die Dialyseflüssigkeit übertreten und den Patienten somit entwässern.

Das Verfahren zur Peritonealdialyse wird in der Regel mit Hilfe von automatischen Peritonealdialysegeräten, wie sie beispielsweise von der Anmelderin unter der Bezeichnung sleep.safe vertrieben werden, durchgeführt.

Dialysegeräte, als Beispiel für komplexe medizinische Fluidmanagementgeräte, haben umfangreiche Funktionen. Um diese Funktionen zu steuern, sind medizinische Fluidmanagementgeräte wie Dialysegeräte mit zumindest einer Steuervorrichtung ausgerüstet. Diese können als CPU (central processing unit) oder Mikrokontroller ausgeführt sein, die von Softwareprogrammen programmiert werden. Die Bedienung solcher Geräte geschieht häufig über Touchscreendisplays. Ein solches Touchscreendisplay kombiniert in einer gemeinsamen Oberfläche eine Ein- und eine Ausgabevorrichtung, in dem es eine berührungsempfindliche Oberfläche bereitstellt, mit der Bedienereingaben detektierbar sind.

Mögliche Ausführungen sehen die räumliche Trennung von Ein- und Ausgabevorrichtung vor, beispielsweise durch ein konventionelles Display, beispielsweise als CRT-Monitor (Cathode Ray Tube), LCD (Liquid Christal Display), Plasma- oder OLED-Display (Organic Light Emitting Device) ausgeführt, als Ausgabevorrichtung und einem davon räumlich getrennten Touchpad, das eine berührempfindliche Oberfläche bereitstellt, mit der Bedienereingaben detektierbar sind, als Eingabevorrichtung.

Unabhängig von der Ausführung der Ein- / Ausgabevorrichtung ist den bisherigen Ausgestaltungen dieser Ein- und Ausgabevorrichtungen gemeinsam, dass sie untrennbar mit dem medizinischen Fluidmanagementgerät verbunden sind. Oftmals bildet die Ein- und Ausgabevorrichtung mit dem medizinischen Fluidmanagementgerät sogar eine bauliche Einheit.

Derartige Ausführungen haben den Nachteil, dass sie teuer sind und einen erhöhten Montage- und Wartungsaufwand nach sich ziehen. So werden oftmals teure großformatige Touchscreendisplays eingesetzt, die bei der Montage des medizinischen Fluidmanagementgeräts zusätzlich einen hohen Aufwand nach sich ziehen.

Medizinische Fluidmanagementgeräte sind oftmals auch mit Sensoren ausgestattet, die Messwerte bezogen auf das Gerät selbst oder der damit ausgeführten Behandlung aufnehmen. Solche Sensoren können beispielsweise optische Sensoren sein, die beispielsweise Füllstände von Gefäßen oder Flussraten von Fluiden überwachen.

Auch die Ausstattung von medizinischen Fluidmanagementgeräten mit Sensoren erweist sich als Kostenfaktor und zieht einen erhöhten Montage- und Wartungsaufwand nach sich. Weiterhin sind speziell ältere Fluidmanagementgeräte in Gebrauch, die kein Display aufweisen, oder nur wenige Sensoren zur Überwachung des Fluidmanagementgeräts oder einer damit ausgeführten Behandlung US2010/137694 offenbart Methoden und eine Vorrichtung, um durch Sensoren erfasste Informationen über den medizinischen Zustand eines Patienten an ein angekoppeltes Datenverarbeitungssystem zu übertragen.

### Beschreibung

Es ist daher die Aufgabe der Erfindung, eine verbesserte Anzeige- und Bedienvorrichtung für medizinische Fluidmanagementgeräte bereitzustellen. Darüber hinaus sollen ein System aus einem medizinischen Fluidmanagementgerät und einer externen Anzeige- und Bedienvorrichtung und ein Verfahren zur Verwendung einer externen Anzeige- und Bedienvorrichtung mit einem medizinischen Fluidmanagementgerät geschaffen werden.

Die Lösung dieser Aufgaben erfolgt erfindungsgemäß durch ein medizinisches Fluidmanagementgerät nach Anspruch 1, durch ein System nach Anspruch 5 sowie durch ein Verfahren nach Anspruch 7.

Vorteilhafte Ausführungsformen sind Gegenstand der Unteransprüche.

Im Folgenden soll die offenbarte Lehre anhand eines Dialysegeräts als ein medizinisches Fluidmanagementgerät erläutert werden. Dem Fachmann ist klar, dass die Erfindung ohne weiteres auf andere medizinische Fluidmanagementgeräte übertragbar ist. Beispiele hierfür sind Infusionsgeräte für medizinische Flüssigkeiten, Herz-Kreislaufunterstützungsgeräte mit extrakorporalem Blutkreislauf, Leberunterstützungsgeräte mit extrakorporalem Blutkreislauf oder Ähnliches. Die Ausführungsformen, die im Folgenden am Beispiel eines Dialysegeräts als Beispiel für ein medizinisches Fluidmanagementgerät beschrieben werden, können im Wesentlichen auf ein anderes medizinisches Fluidmanagementgerät übertragen werden.

Ein Dialysegerät ist oftmals mit einem teuren, relativ großen Display, oftmals auch als Touchscreendisplay ausgeführt, ausgestattet. Um diesen Kostenfaktor einzusparen, wird vorgeschlagen, statt eines aufwändigen Displays nur einfache Anzeigevorrichtungen für elementar wichtige Werte einzubauen. Solche Anzeigevorrichtungen können beispielsweise Siebensegment LED Anzeigen oder LED-Aussteuerungsketten sein.

Ältere Dialysegeräte sind häufig nicht mit einem Display ausgerüstet. Es ist vorstellbar, solche Geräte mit einer erfindungsgemäßen Ankoppelvorrichtung und einer Datenschnittstelle nachzurüsten, um ein externes mobiles Kommunikationsgerät oder einen mobilen Computer mit einer als Display ausgeführten Ausgabevorrichtung als Display zu verwenden.

Der Vorteil einer solchen Umrüstung ist die Verwendung von preiswerten Geräten, wie mobile Kommunikationsgeräte oder mobile Computer, die ohnehin mit einem Display ausgestattet sind, ohne dass größere Umbauarbeiten am Dialysegerät notwendig sind.

Unter mobilen Kommunikationsgeräten werden insbesondere Mobiltelefone oder sogenannte Smartphones verstanden. Smartphones zeichnen sich in der Regel durch ein vergleichsweise großes Touchscreendisplay aus. Darüber hinaus verfügen Smartphones in der Regel über vielseitige Computerfähigkeiten, da sie mit leistungsfähigen Mikroprozessoren und vielseitigen Betriebssystemen ausgerüstet sind. So können Smartphones über Anwendungs- oder Applikationsprogramme, sogenannte Apps, beliebig programmiert werden. Fast immer verfügen Smartphones über einen oder mehrere Sensoren.

So sind Smartphones oftmals mit zumindest einer Kameravorrichtung zur Aufnahme von Fotografien und Videos ausgerüstet. Es sind auch Smartphones mit zwei Kameras an gegenüberliegenden Seiten des Smartphones bekannt.

Darüber hinaus verfügen Smartphones immer über ein Mikrofon, das als akustischer Sensor verwendet werden kann. Ferner können Smartphones mit einer Vielzahl anderer Sensoren ausgestattet sein. Beispiele hierfür sind Neigungssensoren, Erschütterungssensoren oder Temperatursensoren. Ferner fungiert ein Touchscreendisplay als berührempfindlicher Sensor. Ebenso können Fingerabdrucksensoren Bestandteile von Smartphones sein.

Weiterhin sind unter mobilen Computern im Sinne der vorliegenden Offenbarung beispielsweise Laptops, Notebooks oder Tablet-PCs zu verstehen. Die Ausstattung von mobilen Computern und Smartphones kann sich stark ähneln. Sämtliche Ausstattungsmerkmale von Smartphones können auch bei mobilen Computern vorhanden sein. Der wesentliche Unterschied zwischen diesen beiden Gerätekategorien liegt in der Größe des Geräts, insbesondere in der Größe des Displays.

Unabhängig davon, welches Gerät verwendet wird, verfügt das verwendete Gerät über ein Display und eine Schnittstelle zum Datenaustausch mit dem Dialysegerät.

Darüber hinaus weist das mobile Kommunikationsgerät oder der mobile Computer eine Ankoppelvorrichtung auf, über die in Wechselwirkung mit einer entsprechend gestalteten Ankoppelvorrichtung beim Dialysegerät eine mechanische Ankopplung hergestellt werden kann. Die mechanische Ankopplung zwischen mobilem Kommunikationsgerät oder mobilem Computer mit dem Dialysegerät ist vorteilhaft jederzeit im Sinne einer Abkopplung lösbar.

Solche Ankoppelvorrichtungen können umfassen: Steckverbindungen, Haken, Ösen, Klemmen, Magnetverbindungen, Klettverschlüsse oder sonstige Vorrichtungen, die dazu geeignet sind, eine mechanische Verbindung zwischen mobilem Kommunikationsgerät oder dem mobilen Computer, im Folgenden unter dem Begriff "mobiler Computer" zusammengefasst, und dem Dialysegerät herzustellen.

Eine Ankoppelvorrichtung im Sinne der Erfindung ist keine steckbare Kabelverbindung, wie beispielsweise eine RJ45 Netzwerkkabelverbindung. Vielmehr stellt die Ankoppelvorrichtung im Einklang mit der Lehre der vorliegenden Erfindung eine mechanische Verbindung zwischen Dialysegerät und mobilen Computer her, die nicht notwendigerweise auch eine elektrische Verbindung herstellt, und die nicht frei flexibel in alle Raumachsen wie beispielsweise eine Kabelverbindung ist, und das Gewicht des mobilen Computers ohne weitere mechanische Vorrichtung selbstständig trägt. Hierbei kann die relative Position des mobilen Computers zum Dialysegerät einstellbar ausgeführt sein, beispielsweise über Feststellgelenke und/oder mechanische Auszüge. Im Einklang mit der Lehre der vorliegenden Erfindung umfasst der mobile Computer zumindest ein Display, eine Ein- und/oder Ausgabevorrichtung, beispielsweise als Touchscreen ausgebildet, eine Schnittstelle, die zum Datenaustausch mit einem weiteren Gerät ausgebildet ist, eine Steuervorrichtung, beispielsweise eine CPU (central processing unit) bzw. einen Mikrokontroller, sowie eine Ankoppelvorrichtung, die dazu ausgebildet ist, eine mechanische Verbindung mit einem medizinischen Fluidmanagementgerät herstellbar zu machen.

Weiterhin kann im Einklang mit der Lehre der vorliegenden Erfindung der mobile Computer über zumindest einen Sensor verfügen.

Vorteilhaft sind solche Ankoppelvorrichtungen, bei denen eine bauliche Veränderung des externen Geräts nicht notwendig ist. Die Ankoppelvorrichtung am mobilen Computer ist bei dieser Ausführungsform das Gehäuse des mobilen Computers selbst, das in eine entsprechend eingerichtete Ankoppelvorrichtung des Dialysegeräts eingreift. Derartige Ankoppelvorrichtungen des Dialysegeräts können beispielsweise Vertiefungen, wie Schächte sein, in die der mobile Computer gesteckt oder eingeführt wird. Ein anderes Beispiel hierfür ist eine Klemme.

Eine solche Ankoppelvorrichtung kann vorteilhaft so ausgeführt sein, dass eine elektrisch leitende Verbindung zwischen Dialysegerät und mobilen Computer herstellbar ist. Derart ausgestaltet Klemmvorrichtungen können beispielsweise über Stecker oder Buchsen an einer Klemmbacke verfügen, die in ein entsprechend ausgeformtes Gegenstück am mobilen Computer eingreifen und eine elektrische Verbindung mit diesem herstellen. Vertiefungen am Dialysegerät können elektrische Schnittstellen aufweisen, die bei Aufnahme des mobilen Computers mit entsprechenden Schnittstellen am mobilen Computer eine elektrische Verbindung herstellen. Die elektrische Verbindung zwischen Dialysegerät und mobilem Computer kann aber auch durch ein elektrisches Kabel hergestellt werden.

Die Ankoppelvorrichtung des mobilen Computers kann aber auch eine speziell für diesen hergestellte Ankoppelvorrichtung umfassen, die beim mobilen Computer eingebaut werden können. Solche Ankoppelvorrichtungen sind beispielsweise Steck- oder Schraubverbindungen, gegebenenfalls dreh- oder schwenkbar gelagert oder ausziehbar, die den mobilen Computer mit dem Dialysegerät verbindbar machen.

In vorteilhafter Weise kann bei einer elektrischen Verbindung zwischen Dialysegerät und mobilem Computer eine Energieübertragung in Form elektrischer Energie vom Dialysegerät zum mobilen Computer stattfinden. So kann das Dialysegerät die Stromversorgung für den mobilen Computer bereitstellen bzw. die häufig im mobilen Computer vorhandenen Akkumulatorbatterie aufladen.

Gemäß der Lehre der vorliegenden Offenbarung kann der Datenaustausch zwischen Dialysegerät und mobilem Computer aber auch drahtlos erfolgen. Gängige drahtlose Datenübertragungswege sind hierbei eine Funkübertragung wie WLAN und Bluetooth, oder auch Infrarotdatenübertragung.

Bei der drahtlosen Übertragung ist die sichere Zuordnung zwischen mobilem Computer und Dialysegerät wesentlich. Daher ist gemäß der Lehre der vorliegenden Offenbarung eine Vorrichtung vorgesehen, die anzeigt, dass ein mobiler Computer sich in der Ankoppelvorrichtung befindet.

Eine solche Vorrichtung kann einen Schalter oder Taster umfassen, der bei der Ankopplung des mobilen Computers betätigt wird. Über eine Abfrage des Schaltzustands durch das Dialysegerät ist somit erkennbar, ob ein mobiler Computer angekoppelt ist.

Weiterhin sieht die Lehre der vorliegenden Offenbarung vor, die Authentizität zwischen mobilem Computer und Dialysegerät sicher zu stellen, so dass der Datenaustausch zwischen Dialysegerät und mobilem Computer mit den dafür vorgesehenen Geräten geschieht, und nicht ungewollt mit anderen sich in der Nähe befindlichen Geräten.

Eine derartige Authentifizierung mit anschließender Kopplung und Initialisierung der drahtlosen Datenübertragung zwischen beiden Geräten kann z.B. über eine Nahfeld-Funktechnologie wie NFC (Near field communication) erfolgen.

Bei allen möglichen Ausführungsbeispielen werden für die Übertragung von Daten solche Datenübertragungsprotokolle bevorzugt, die sicher bezüglich der Vertraulichkeit, der Authentizität und Integrität sind, wie beispielsweise die Datenübertragungsprotokolle IPSec ( Internet Protocol Security) oder VPN (Virtual Private Network) oder OpenVPN.

Weiterhin kann zur Authentifizierung des Dialysegeräts und des mobilen Computers ein Datenübertragungsprotokoll mit sicherer Identifikation der sich austauschenden Geräte, wie beispielsweise Bluetooth oder IrDA (Infrared Data Association) verwendet werden.

Die Erfindung sieht in einer Ausführungsform vor, dass das Dialysegerät Informationen zum mobilen Computer überträgt, die auf dem Display des mobilen Computers zur Anzeige von Daten führen, die das Dialysegerät und/oder eine mit dem Dialysegerät durchgeführte Behandlung betreffen. Der solchermaßen übertragene Bildinhalt entspricht hierbei in einer Ausführungsform dem eines ansonsten baugleichen Dialysegeräts, welches aber mit einem integrierten Display ausgestattet ist. Auf diese Weise kann die Displayfunktionalität an den mobilen Computer umgeleitet werden.

Es kann aber auch vorgesehen sein, dass der übertragene Bildinhalt von den Displayeigenschaften des mobilen Computers abhängt. So kann der Übersichtlichkeit halber der Bildschirminhalt bei mobilen Computern mit vergleichsweise kleineren Displays, wie beispielsweise Smartphones gegenüber mobilen Computern mit großen Displays, wie beispielsweise Tablet-PCs reduziert werden.

Die Information, welche Displaygröße und Auflösung ein mobiler Computer hat, kann dazu beim initialen Verbinden des mobilen Computers an das Dialysegerät übertragen werden.

In einer alternativen Ausführungsform passt ein entsprechend programmierter mobiler Computer die Anzeige der empfangenen Bildinformation selbstständig seinen Displayeigenschaften an.

In einer alternativen Ausfuhrungsform kann die Eingabevorrichtung des mobilen Computers, beispielsweise als Touchscreen ausgeführt, zur Bedieneingabe am Dialysegerät verwendet werden. Hierbei werden die an der Eingabevorrichtung des mobilen Computers vorgenommenen Bedieneingaben über die Schnittstelle an das Dialysegerät weiter gegeben.

Eine weitere alternative Ausführungsform sieht vor, die Bedienvorrichtungen am Dialysegerät für Bedieneingaben zu sperren, sobald der mobile Computer zur Bedienung des Dialysegeräts angekoppelt ist, um Doppeleingaben zu vermeiden.

Um die Reinigung des Dialysegeräts zu erleichtern, kann vorgesehen sein, dass die Ankoppelvorrichtung derart in das Dialysegerät einklappbar ist, dass sich im eingeklappten Zustand bevorzugt eine ebene, nicht unterbrochene Gehäuseoberfläche des Dialysegeräts ausbildet.

Ein weiterer Aspekt betrifft die Nutzung von Sensoren von mobilen Computern zum Zweck der Überwachung des Dialysegeräts. Smartphones und Tablet-PCs beispielsweise sind häufig mit zumindest einer Kameravorrichtung zur Aufnahme von Fotografien und Videos ausgerüstet. Es sind auch Ausführungen mit zwei Kameras an gegenüberliegenden Seiten des Geräts bekannt.

Darüber hinaus verfügen Smartphones und Tablet-PCs häufig über ein Mikrofon, das als akustischer Sensor verwendet werden kann. Ferner können Smartphones und Tablet-PCs mit einer Vielzahl anderer Sensoren ausgestattet sein. Beispiele hierfür sind Neigungssensoren, Erschütterungssensoren oder Temperatursensoren. Ferner fungiert ein Touchscreendisplay als berührempfindlicher Sensor. Ebenso können Fingerabdrucksensoren Bestandteile von Smartphones und Tablet-PCs sein.

Ein Ausführungsbeispiel der Erfindung sieht vor, dass der mobile Computer derart mechanisch und elektrisch an das Dialysegerät ankoppelbar ist, dass eine Kamera des mobilen Computers auf das Dialysegerät oder Teile des Dialysegeräts gerichtet ist.

Über eine entsprechende Programmierung kann der mobile Computer das Dialysegerät überwachen und/oder steuern. So ist es beispielsweise möglich, die Pumpenrate von Schlauchrollenpumpen zu überwachen, dadurch, dass über eine Videoauswertung im mobilen Computer die Bewegung des Rotors der Schlauchrollenpumpe erfasst und ausgewertet wird. Für die Realisation der Videoauswertung ist der mobile Computer entsprechend programmiert.

Weiterhin können beispielsweise Füllstände in Tropfkammern in ähnlicher Weise erkannt werden. Auch die Überwachung von anderen medizinischen Fluiden, wie beispielsweise angehängten Beuteln mit Infusionslösungen, ist möglich. So kann der mobile Computer beispielsweise eine Warnmeldung ausgeben, wenn eine vorgehaltene Infusionslösung droht leer zu laufen.

Ferner ist es auch möglich, über die Auswertung des Kamerasignals Störungen im Betrieb zu erkennen. Beispielsweise kann ein Leck im extrakorporalen Blutkreislauf erkannt werden, da austretendes Blut durch die Kameraauswertung erkannt werden kann. In einem solchen Fall kann eine Alarmmeldung vom mobilen Computer ausgegeben werden und/oder Steuersignale zum Dialysegerät gesendet werden, die zur Folge haben, dass die Dialysebehandlung unterbrochen und das Dialysegerät in einen sicheren Zustand versetzt wird, beispielsweise durch das Unterbrechen der Blutförderung durch die Blutpumpe und das Abklemmen der arteriellen und venösen Patientenzugänge durch entsprechende Schlauchklemmen. In ähnlicher Weise kann der mobile Computer in die Steuerung der Dialysemaschine auch abhängig anderer Sensorwerte eingreifen.

Weiterhin können durch die Kamera- bzw. Videoauswertung im mobilen Computer die verwendeten Zubehörteile erkannt werden. So können beispielsweise der Dialysefilter, oder eingelegte Schlauchsets anhand ihrer optischen Eigenschaften erkannt werden.

Der mobile Computer kann hierdurch beispielsweise erkennen, ob ein zum Dialysegerät passender oder vom Hersteller des Dialysegeräts zur Verwendung zugelassener Dialysefilter oder sonstige Zubehörteile verwendet werden und abhängig von dieser Überprüfung eine Warnung aussprechen, sollte ein nicht passendes oder nicht zugelassenes Zubehörteil verwendet werden.

In diesem Fall kann der mobile Computer einen solchen Vorgang protokollieren, beispielsweise in einem geräteinternen Speicher, oder durch eine Datenübertragung an ein weiteres Gerät. Diese Datenübertragung kann beispielsweise als Email, SMS oder Pagernachricht an eine übergeordnete Instanz, beispielsweise den Stationsarzt, erfolgen. Hierfür kann eine weitere Schnittstelle des mobilen Computers, wie beispielsweise eine Mobilfunkvorrichtung verwendet werden.

Eine Ausführungsform sieht vor, dass eine Kamera des mobilen Computers auf den Patienten gerichtet ist. Dieses dient zum einen zur Überwachung des Patienten, wenn das Bild der Kamera beispielsweise über eine Datenübertragung an weitere Geräte für medizinisches Personal übertragen wird, zum anderen können über eine Videoauswertung im externen Gerät bestimmte Gesten des Patienten erkannt werden, die in mit der jeweiligen Geste verknüpften Aktionen münden. So kann eine bestimmte Geste des Patienten, z.B. ein nach unten gedrehter Daumen, signalisieren, dass der Patient wünscht, die Lautstärke von Tonmeldungen des Dialysegerätes zu reduzieren, was dann durch die Datenübertragung vom mobilen Computer zum Dialysegerät initiiert wird.

Die Überwachung des Dialysegeräts mit einer Kamera des mobilen Computers erfolgt hierbei auf Basis bekannter Verfahren der Bild- bzw. Videoauswertung. Die WO2012006896A1 beispielsweise beschreibt ein Verfahren zur Auswertung des Kamerasignals einer auf eine Tropfkammer gerichteten Kamera, wobei die Tropfgeschwindigkeit, als auch der Flüssigkeitspegel in der Tropfkammer bestimmt wird. Grundsätzlich sind dynamische Prozesse, die veränderliche Ansichten nach sich ziehen, wie beispielsweise das Pumpen von Flüssigkeiten durch sich bewegende Pumpen, wie Schlauchrollenpumpen, durch eine Kameraabtastung überwachbar, wenn bekannte Verfahren zur Bild- bzw. Videoauswertung zur Anwendung kommen.

Eine weitere Ausführungsform sieht vor, dass dem mobilen Computer die Art der anstehenden Behandlung bekannt gemacht wird. Dies kann durch das Dialysegerät selbst erfolgen, in dem es eine entsprechende Datenübertragung an den mobilen Computer veranlasst. Diese Information kann dem mobilen Computer aber auch durch Eingabe am mobilen Computer selbst, oder über andere Datenübertragungswege bekannt gemacht werden. Wesentlich ist, dass dem mobilen Computer sowohl das Dialysegerät, als auch die anstehende Behandlung bekannt ist.

Mit dieser Information kann durch eine entsprechende Programmierung, die auf hinterlegte Daten für die Art und Weise der Aufrüstung des Dialysegeräts für eine bestimmte Behandlung zurückgreift, der Vorgang der Aufrüstung überwacht und angeleitet werden.

Diese Überwachung kann sowohl die Struktur, als auch den Aufrüstvorgang selbst überwachen. Es ist beispielsweise bei Dialysebehandlungen notwendig, den Dialysefilter und den extrakorporalen Blutkreislauf vollständig zu entlüften. Der Dialysefilter ist beispielsweise bei der Entlüftung oftmals zu einem bestimmten Zeitpunkt um 180 Grad zu drehen. Auch dieser Vorgang kann durch die Kameraauswertung überwacht werden.

Vorteilhafter Weise kann der mobile Computer dem Anwender optische und/oder akustische Hinweise geben, wie die Aufrüstung im konkreten Fall zu geschehen hat. Dies kann über das Display des mobilen Computers erfolgen. Akustische Hinweise können über einen Lautsprecher des mobilen Computers ausgegeben werden, etwa unter Verwendung eines Sprachsynthetisierers.

In gleicher Weise kann auch die Abrüstung der Maschine überwacht werden. Beispielsweise sind bei einer Dialysebehandlung bestimmte Schritte notwendig, um einen Patienten von dem Dialysegerät nach erfolgter Behandlung zu trennen. Auch dieser Vorgang kann überwacht und/oder über die Ausgabevorrichtungen des mobilen Computers angeleitet werden.

Auch sonstige Sensoren des mobilen Computers können zur Überwachung verwendet werden. In einer Ausführungsform ist der mobile Computer mit einem Temperatursensor ausgerüstet, beispielsweise mit einer Infrarotkamera. Diese kann in der schon beschriebenen Weise dazu verwendet werden, um das Dialysegerät und eine damit ausgeführte Behandlung zu überwachen. Im Falle einer Infrarotkamera ist auch vorstellbar, dass alternativ oder zusätzlich auch der Patient überwacht wird, und beispielsweise die Körpertemperatur des Patienten gemessen wird. Über- oder Untertemperaturen des Patienten können so berührungsfrei erkannt werden, und entsprechende Hinweise oder Warnmeldungen über die Ausgabevorrichtungen des mobilen Computers ausgegeben werden.

Auch kann eine akustische Überwachung des Dialysegeräts, der damit ausgeführten Behandlung oder des Patienten erfolgen, in dem ein Mikrofonsignal des mobilen Computers entsprechend ausgewertet wird.

So erzeugt ein spezifisches Dialysegerät mit spezifischer Aufrüstung während einer spezifischen Behandlung auch eine spezifische Geräuschkulisse, die durch das vorhandene Mikrofon des mobilen Computers erfassbar ist. Eine entsprechende Auswertung des Mikrofonsignals, die über eine entsprechende Programmierung des mobilen Computers erfolgt, kann hierbei Abweichungen vom erwarteten Geräuschsignal erkennen und in diesem Fall eine Meldung über die Ausgabevorrichtungen des mobilen Computers abgeben. Zur besseren Auswertung kann vorgesehen sein, dass Fremdgeräusche, wie sie innerhalb einer Behandlung häufig vorkommen, durch signaltechnische Bearbeitung des Mikrofonsignals herausgefiltert werden.

Ein weiterer Aspekt der akustischen Überwachung betrifft den Patienten selbst, dessen akustische Äußerungen über das Mikrofon des mobilen Computers erkennbar sind. So können beispielsweise unnormale Geräusche (Röcheln, Klagen) als auch Willensäußerungen des Patienten erkannt werden und weiter Schritte, wie beispielsweise Alarmmeldungen, die per Datenübertragung auch zu weiteren Geräten übermittelbar sein können, ausgegeben werden. Auch alle weiteren Sensoren, mit denen der mobile Computer ausgerüstet ist, können zur Überwachung des Dialysegeräts, der Dialysebehandlung oder des Patienten eingesetzt werden.

So kann beispielsweise ein vorhandener Fingerabdruckscanner des mobilen Computers dazu verwendet werden, den Patienten eindeutig zu identifizieren. Sind die Behandlungsdaten und der Patient dem mobilen Computer bekannt gemacht worden, kann beispielsweise die Aufrüstung des Dialysegeräts basierend auf den bekannten Behandlungsdaten dann mit Hilfe einer vorhandenen Kamera überwacht werden.

Unabhängig davon, mit welchen Sensoren der mobile Computer ausgerüstet ist, können diese dazu verwendet werden, den Patienten zu überwachen und/oder das Dialysegerät bzw. die Dialysebehandlung zu überwachen und/oder zu steuern.

Im Einklang mit der Lehre der vorliegenden Erfindung ist hierzu vorgesehen, dass der mobile Computer derart an das medizinische Fluidmanagement ankoppelbar ist, dass der entsprechende Sensor ein Signal erfassen kann, das auf das medizinische Fluidmanagementgerät und/oder auf eine mit dem medizinischen Fluidmanagementgerät durchgeführte Behandlung bezogen ist. Die Steuervorrichtung des mobilen Computers ist entsprechend dazu eingerichtet, abhängig vom erfassten Signal Steuersignale an das medizinische Fluidmanagementgerät weiter zu geben und/oder Alarmmeldungen auszugeben. Einen weiteren Vorteil im Einklang mit der Lehre der vorliegenden Erfindung betrifft die Möglichkeit, dass der mobile Computer Behandlungsdaten über mehrere Behandlungen abspeichert und dem Bediener zugänglich macht. Auf diese Weise wird eine Behandlungshistorie gebildet, die für den behandelnden Arzt wertvolle Informationen beinhaltet, anhand deren er die zukünftige Behandlung ausrichten kann. Oftmals sind nämlich in konventionellen Dialysegeräten nur begrenzte Speichermöglichkeiten für das Abspeichern von Behandlungsdaten vorhanden. Durch die Verwendung eines mobilen Computers kann dieser Nachteil umgangen werden. Der interne Speicher von mobilen Computern ist oftmals umfangreicher als der von Dialysegeräten. Die Speicherkapazität von mobilen Computern kann auch dadurch erweitert werden, in dem diese auf weitere externe Geräte, beispielsweise ein Netzwerk wie beispielsweise einen Cloud-Service, zurückgreifen.

Weiterhin kann der mobile Computer auch dazu benutzt werden, Berechnungen durchzuführen, für welche das Dialysegerät nicht eingerichtet ist. Dies kann beispielsweise dann der Fall sein, wenn das Dialysegerät nicht über die entsprechende Programmierung verfügt, oder die dem Dialysegerät unterliegende Hardware nicht dazu in der Lage ist, die entsprechende Berechnung durchzuführen, weil sie beispielsweise veraltet ist, oder die für eine Berechnung notwendigen Sensordaten, die vom mobilen Computer aufgenommen werden, nicht zur Verfügung stehen. Für derartige Berechnung ist der mobile Computer über jeweils spezifische Computerprogramme programmiert.

Generell verfügen der mobile Computer und das medizinische Fluidmanagementgerät im Einklang mit der Lehre der vorliegenden Erfindung über spezifische Programmierungen, die die Bedienung des medizinischen Fluidmanagementgerätes oder die Anzeige von Daten das medizinische Fluidmanagementgerät oder einer damit ausgeführten Behandlung und/oder die Verwendung von Sensoren zur Überwachung und Steuerung des medizinischen Fluidmanagementgerätes oder einer damit ausgeführten Behandlung betreffend entsprechend steuern und die beschriebenen Verfahren ausführen.

Diese spezifischen Programmierungen sind Computerprogramme und können direkt in einen internen Speicher des mobilen Computers und/oder des medizinischen Fluidmanagementgerätes geladen werden und Softwarecodeabschnitte umfassen, die die beschriebenen Verfahren ausführen, wenn die Programme auf dem mobilen Computer oder dem medizinischen Fluidmanagementgerät laufen. Die Computerprogramme können als Computerprogrammprodukte, die computerlesbare Programmmittel umfassen, auf Datenmedien vorgehalten werden. Diese Datenmedien sind in einem Computer einsetzbar und umfassen neben physikalischen Speichern, wie Disketten, CD-Roms, Speicherkarten, USB Sticks oder DVDs auch die Speicherung innerhalb von Netzwerken, wie dem Internet, auf die der Benutzer Zugriff haben kann.

Weiterhin können im Einklang mit der Lehre der vorliegenden Erfindung alle im mobilen Computer vorhandenen Vorrichtungen und Verfahren in vorteilhafter Weise dazu verwendet werden, die Funktionalität eines medizinischen Fluidmanagementgeräts, insbesondere eines Dialysegeräts, zu ergänzen bzw. zu erweitern.

Beispiele hierfür sind:
Der mobile Computer kann als Netzwerkzugang bzw. zur Verbindung mit dem Internet genutzt werden, in dem die entsprechenden Datenverbindungen, die im mobilen Computer etablierbar sind, über eine Datenschnittstelle an das Dialysegerät weitergeleitet werden. Eine solche Datenanbindung über den mobilen Computer wird auch Tethering genannt.

Über eine so ermöglichte Internetverbindung kann auch ein Therapievergleich für verschiedene Patienten erfolgen. Hierzu werden die aktuellen Behandlungsparameter mit den aktuellen Therapien einer Patientengemeinschaft bevorzugt anonym verglichen und bewertet. Bewertungsgrundlagen können beispielsweise umfassen: Größe, Gewicht, Rasse/Ethnik und Blutwerte, sowie Gerätetyp und die damit durchgeführte Behandlung bzw. Therapie.

Oftmals sind mobile Computer auch mit einer Vorrichtung zur Bestimmung des Standortes des Gerätes ausgerüstet, beispielsweise durch einen GPS-Sensor (Global Positioning System) oder über die Auswertung lokaler Netzwerkverbindungen. Diese Information kann im Einklang mit der Lehre der vorliegenden Erfindung auch vorteilhaft dazu benutzt werden, das Dialysegerät, oder eine damit ausgeführte Therapie zu beeinflussen.

Beispielsweise kann anhand der Information über den Standort des Dialysegeräts eine Aussage über die an diesem Standort übliche Wasserqualität abgeleitet werden, was Einfluss auf die Herstellung von Lösungen haben kann, die mit dem Dialysegerät hergestellt werden.

Weiterhin kann anhand der Information über den Standort des Dialysegeräts auch erkannt werden, ob an diesem Standort bestimmte Therapieoptionen erlaubt sind bzw. von den lokalen Gesundheitskassen bezahlt werden. Eine Sperrung dieser Therapieoption kann dementsprechend veranlasst werden. Alternativ oder ergänzend kann eine Behandlungsoption auch frei gegeben werden, wenn die Bezahlung dieser Option sichergestellt wurde. Dieses kann beispielsweise auch durch eine entsprechende Applikation im mobilen Computer geschehen, z.B. über die Eingabe von Kreditkartendaten. Bezahlverfahren, die eine Internetverbindung nutzen, können ebenso angewandt werden. Diese sind unter dem Begriff E-Commerce (electronic commerce) bekannt.

Vielen Ausführungsformen ist gemeinsam, dass der mobile Computer zumindest mit einem anderen Gerät als mit dem medizinischen Fluidmanagementgerät kommuniziert. Dieses andere Gerät kann beispielsweise ein weiterer Computer, beispielsweise als Server ausgeführt, ein Pager oder eine Mobilfunkeinrichtung sein.

Die vorliegende Offenbarung umfasst die Verwendung mobiler Computer als Ein- oder Ausgabevorrichtung, sowie als Steuer- und/oder Überwachungsvorrichtung und die Verwendung von Verfahren und Vorrichtungen von mobilen Computern, beispielsweise von Sensorvorrichtungen, bei medizinischen Fluidmanagementgeräten und mit diesen durchgeführten Behandlungen.

### Kurze Beschreibung der Figuren

Weitere Einzelheiten und Vorteile im Einklang mit der Lehre der vorliegenden Erfindung werden anhand von in den Zeichnungen dargestellten Ausführungsbeispielen näher beschrieben. Es zeigen:
Die Figur 1 ein konventionelles medizinisches Fluidmanagementgerät, beispielhaft als Hämodialysemaschine ausgebildet;
die Figur 2 ein medizinisches Fluidmanagementgerät im Einklang mit der Lehre der vorliegenden Erfindung, beispielhaft als Hämodialysegerät ausgebildet, mit einem als Tablet-PC ausgeführten mobilen Computer, der als Display dient;
die Figur 3 eine Detailansicht des als Tablet-PC ausgeführten mobilen Computers aus der Figur 2;
die Figuren 4a, 4b und 4c ein konventionelles medizinisches Fluidmanagementgerät, beispielhaft als Peritonealdialysegeräte ausgebildet, sowie im Einklang mit der Lehre der vorliegenden Erfindung mit einem Smartphone bzw. einem Tablet-PC, die jeweils als Display für das Peritonealdialysegerät dienen;
die Figur 5 ein Blockdiagramm eines Systems aus medizinischem Fluidmanagementgerät und mobilem Computer im Einklang mit der Lehre der vorliegenden Erfindung;
die Figur 6 ein Flussdiagramm eines Verfahrens im Einklang mit der Lehre der vorliegenden Erfindung und
die Figur 7 ein Flussdiagramm eines anderen Verfahrens im Einklang mit der Lehre der vorliegenden Erfindung.

### Ausführliche Beschreibung der Figuren

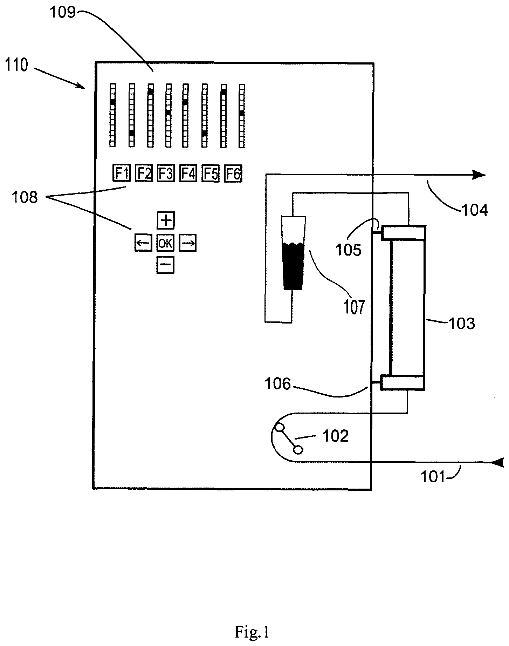

Die Figur 1 zeigt ein als Hämodialysegerät ausgeführtes medizinisches Fluidmanagementgerät, welches nicht mit einem eigenen Display ausgerüstet ist. Solche Dialysegeräte waren vor allem in der Vergangenheit weit verbreitet und werden auch heute noch eingesetzt.

Die Dialysemaschine 110 zeigt schematisch Teile eines extrakorporalen Blutkreislaufs mit einer arteriellen Blutleitung 101, die Blut eines Patienten (nicht dargestellt) ableitet. Die Blutpumpe 102 fördert das Blut durch einen Dialysefilter 103, der mit einer semipermeablen Membran ausgestattet ist, die den extrakorporalen Blutkreislauf von einem Dialysatkreislauf semipermeabel trennt. Über die venöse Leitung 104 wird das behandelte Blut dem Patienten zurückgegeben, nachdem in der venösen Tropfkammer 107 eventuell vorhanden Luftblasen aus dem Blut abgeschieden wurden. Über die Dialysatleitungen 105 und 106 wird Dialysat durch den Dialysefilter 103 gepumpt, wo es über die semipermeable Membran des Dialysefilters 103 zu einem diffusiven Stoffaustausch mit dem Blut des Patienten kommt. Wird zusätzlich ein Druckgradient von der Blutseite des Dialysefilters zur Dialysatseite des Patienten aufgebaut, wird Plasmawasser aus dem Blut in das Dialysat abgepresst. Das Blut des Patienten kann so entwässert werden. Das Dialysat wird in dem Hämodialysegerät 110 hergestellt und nach Gebrauch verworfen. Es können Vorrichtungen beim Hämodialysegerät vorgesehen sein, die dem Patientenblut eine sterile Substitutionslösung zuführen. Dieses kann stromaufwärts des Dialysefilters passieren, wobei man von Prädilution spricht, oder stromabwärts des Dialysefilters, was Postdilution genannt wird.

Der Füllstand des Blutes in der venösen Tropfkammer kann gegebenenfalls durch verschiedene Sensoren des Dialysegeräts überwacht werden.

Das beispielhafte Dialysegerät 110 ist mit einer Reihe von LED-Leisten 109 als Anzeigevorrichtung für wichtige Messwerte wie Blutdruck, Ultrafiltrationsrate, Substitutionsrate, Blutflussrate, Dialysatflussrate usw. ausgerüstet. Durch die begrenzte Anzahl der LEDs in einer solchen LED-Leiste ist deren Auflösung aber eingeschränkt.

Alternativ und nicht in Figur 1 gezeigt, können beispielsweise auch Siebensegment LED Anzeigen verwendet werden.

Über die Bedientasten 108 können Bedienereingaben am Dialysegerät vorgenommen werden. Im Einklang mit der vorliegenden Offenbarung können derartige Dialysegeräte mit einer Ankoppelvorrichtung für einen mobilen Computer nachgerüstet werden. Hierbei wird auch eine Datenschnittstelle zum mobilen Computer ausgebildet. Vorteilhaft kann auch eine Energieübertragung vom Dialysegerät zum mobilen Computer eingerichtet werden.

Auf diese Weise können vorhandene, wenig komfortable Dialysegeräte preisgünstig so nachgerüstet werden, dass eine komfortable Bedienung ermöglicht wird.

Als Alternative können Dialysegeräte hergestellt werden, die keine oder nur kleine und/oder preiswerte Displays aufweisen. Solche Dialysegeräte sind vorteilhaft in Bezug auf die Kosten, der Fertigung, der Wartung und/oder der Entwicklung. Häufig verfügt der Benutzer von Dialysegeräten bereits über entsprechende mobile Computer, die für diverse Aufgaben am Dialysegerät verwendet werden können.

Einen weiteren Vorteil im Einklang mit der Lehre der vorliegenden Erfindung stellt die Möglichkeit dar, einen einzelnen mobilen Computer an mehrere unterschiedliche medizinische Fluidmanagementgeräte ankoppeln zu können. So kann beispielsweise der behandelnde Arzt oder sonstiges medizinisches Personal innerhalb einer Dialysestation, die mit Dialysegeräten, die keine oder nur kleine und/oder preiswerte Displays aufweisen, einen einzelnen mobilen Computer vorhalten und diesen jeweils an jenes Dialysegerät koppeln, mit welchem sie gerade interagieren möchten. Auf diese Weise lassen sich die Kosten innerhalb einer Dialysestation noch weiter senken.

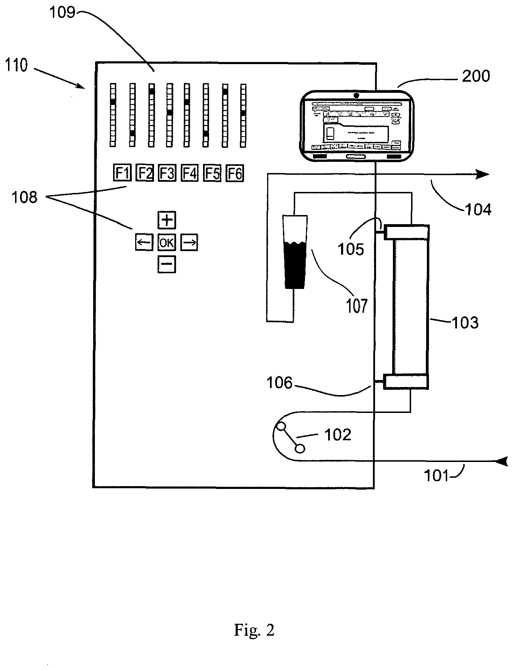

Die Figur 2 zeigt ein medizinisches Fluidmanagementgerät, beispielhaft als Hämodialysegerät ausgebildet, mit einem als Tablet-PC ausgeführten mobilen Computer, der als Display dient. Das Dialysegerät entspricht in seinen dialysebezogenen Funktionen im Wesentlichen dem Aufbau des im Zusammenhang mit der Figur 1 beschriebenen Hämodialysegerätes, auf dessen Beschreibung Bezug genommen wird an Stelle einer Wiederholung. Gleiche Bezugszeichen entsprechen gleichen oder entsprechenden Elementen. Ergänzend wird ein handelsüblicher Tablet-PC 200 verwendet, der am Dialysegerät 110 in nicht näher gezeigter Weise angekoppelt wird. Für die Ankopplung des Tablet-PCs 200 an das Hämodialysegerät 110 sind vielfältige Vorrichtungen geeignet. Beispielsweise kann eine Klemme am Hämodialysegerät befestigt werden, die den Tablet-PC festklemmt. Eine solche Lösung hat den Vorteil, dass keine baulichen Eingriffe am Tablet-PC notwendig sind. Die Ankoppelvorrichtung ist vorteilhaft so ausgebildet, dass verschiedene externe Geräte, wie Smartphones oder Tablet-PCs ankoppelbar sind. Dies kann beispielsweise über verschiebbare Klemmvorrichtungen realisiert werden, die sich den Abmessungen der externen Geräte anpassen lassen. Die Ankopplung des mobilen Computers kann derart erfolgen, dass vorhandene Sensoren des mobilen Computers Messwerte, die das Dialysegerät oder eine mit dem Dialysegerät durchgeführte Behandlung betreffen, aufnehmen können.

Gemäß der bevorzugten Ausführungsform ist die Ankoppelvorrichtung derart ausgebildet dass der mobile Computer in einigem Abstand vom Dialysegerät befestigt wird, um beispielsweise einer Kamera am Rücken des externen Geräts die Sicht beispielsweise auf den extrakorporalen Blutkreislauf oder Teile des extrakorporalen Blutkreislaufs zu ermöglichen. Die bevorzugte beanspruchte Ankoppelvorrichtung ist ausziehbar, dreh- oder schwenkbar, so dass der mobile Computer in jeder gewünschten Lage zum Dialysegerät ankoppelbar ist.

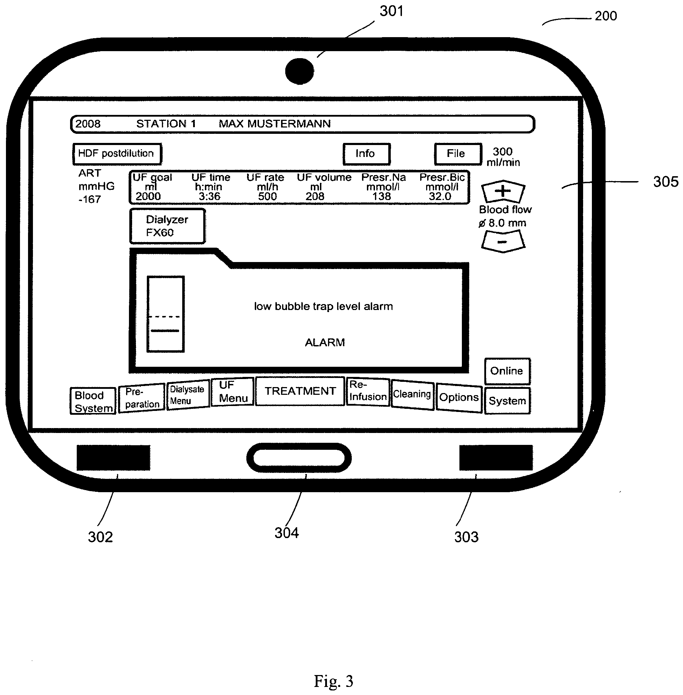

Die Figur 3 zeigt den als Tablet-PC ausgeführten im Zusammenhang mit Figur 2 beschriebenen mobilen Computer 200 im Detail mit einem typischen Displayinhalt 305. Der Tablet-PC 200 ist in diesem Beispiel mit einer Kamera 301 auf der Frontseite des Displays ausgerüstet, sehr häufig sind solche Tablet-PCs auch auf der Rückseite mit einer Kameravorrichtung ausgerüstet. Weiterhin umfasst der Tablet-PC 200 zwei Lautsprecher 302 und 303 und ein Mikrofon 304 als weitere Ein- und Ausgabevorrichtungen. Der Displayinhalt 305 entspricht im Wesentlichen dem für Hämodialysemaschinen, die mit einem integrierten Display ausgestattet sind (oftmals auch als Touchscreen ausgeführt). Die Information, die hierbei anzeigt wird, ist wesentlich umfangreicher und detaillierter, als die Information, die ein Hämodialysegerät ohne Display, wie in Figur 1 gezeigt, anzeigen kann. Sie umfasst beispielsweise den Namen der Maschine (2008, Station 1), den Namen des Patienten (MAX MUSTERMANN), die Art der Behandlung (HDF postdilution für Hämodiafiltration mit Postdilution) und vielfältige Mess- und Einstellwerte.

Ferner wird im Beispiel der Figur 3 auf dem Display auch eine Alarmmeldung ausgegeben, die auf einen zu niedrigen Blutlevel in der venösen Tropfkammer aufmerksam machen soll. Hierbei kann der alarmauslösende Zustand vom Tablet-PC selbst erkannt werden, beispielsweise in dem eine rückseitig am Tablet-PC angebrachte Kamera den Blutlevel in der venösen Tropfkammer optisch überwacht.

Der Alarmzustand kann aber auch durch die Datenübertragung vom Dialysegerät zum Tablet-PC übermittelt werden. Die Alarmmeldung kann dann im Klartext auf dem Display ausgegeben werden. Dies stellt einen Vorteil dar gegenüber einer Alarmanzeige bei einem Dialysegerät ohne eigenes Display, die oftmals nur symbolisch ist und vom Bedienpersonal richtig interpretiert werden muss.

Die Erfindung trägt also auch zu einem schnellen und sicheren Erkennen von Alarmzuständen bei.

Weiterhin kann auch die Bedienung des Dialysegeräts dadurch komfortabler und einfacher gestaltet werden, in dem hierfür das häufig vorhandene Touchscreendisplay des mobilen Computers verwendet wird. Das Ein- und Ausgabekonzept von modernen Dialysegeräten mit integriertem Touchscreendisplay kann auf diese Weise auch auf vorhandene einfachere oder neu hergestellte einfache und preiswerte ausgestattete Dialysegeräte übertragen werden, in dem mobile Computer als vollständiger Ersatz für ein Touchscreendisplay verwendet werden.

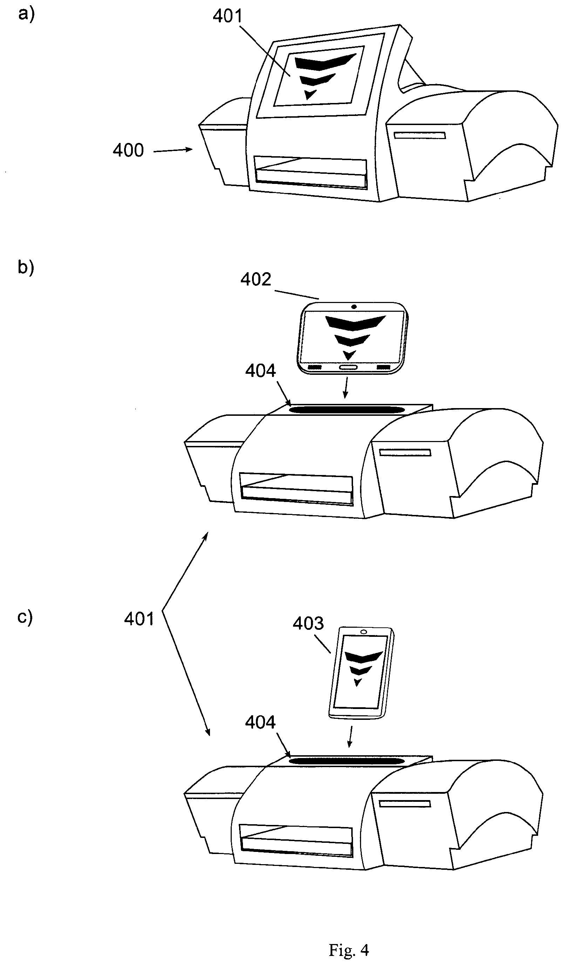

Die Figuren 4b und 4c zeigen jeweils Ausführungsformen der Erfindung für Peritonealdialysegeräte. Das Peritonealdialysegerät 400, das in Figur 4a gezeigt wird, ist auf konventionelle Art ausgeführt. Es weist ein integriertes Display 401 auf.

Die beiden Dialysegeräte 401 der Figuren 4b und 4c sind mit einer Ankoppelvorrichtung 404 ausgerüstet und weisen kein eigenes Display auf. Die Ankoppelvorrichtungen in den Figuren 4b und 4c sind exemplarisch als Schacht zur Aufnahme eines mobilen Computers ausgeführt.

Vorstellbar ist, dass der Schacht ferner auch elektrische Steckverbindungen aufweist, die mit entsprechenden Schnittstellen am mobilen Computer verbindbar sind.

Die Figur 4b zeigt eine Ausführungsform mit einem Tablet-PC 402 als mobiler Computer und die Figur 4c eine Ausführungsform mit einem Smartphone 403 als mobilen Computer. In den Figuren 4b und 4c ist das Peritonealdialysegerät mit einer Ankoppelvorrichtung 404 ausgerüstet, die universell zur Ankopplung mehrerer Geräte geeignet ist. Denkbar sind auch jegliche andere Ankoppelvorrichtungen, die in schon beschriebener Weise ausgeführt sein können.

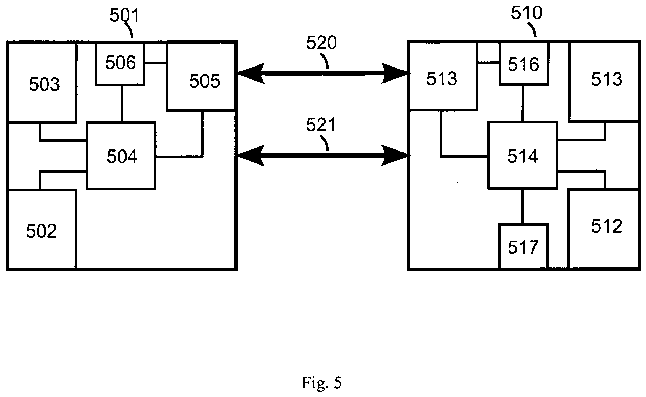

Die Figur 5 zeigt schematisch ein medizinisches Fluidmanagementgerät 501 und einen mobilen Computer 510 im Einklang mit der Lehre der vorliegenden Erfindung. Das medizinische Fluidmanagementgerät 501 verfügt über eine Eingabevorrichtung 502 und vorteilhaft über eine Ausgabevorrichtung 503. Über die Eingabevorrichtung 502 kann der Benutzer Bedieneingaben vornehmen. Die Eingabevorrichtung kann Tasten, Schalter oder Tastaturen umfassen. Die Ausgabevorrichtung 503 gibt Informationen, das medizinischen Fluidmanagementgerät und/oder eine Behandlung, die mit dem medizinischen Fluidmanagementgerät durchgeführt wird, betreffend, aus. Vorteilhaft ist die Ausgabevorrichtung als Display ausgeführt. Im Einklang mit der Lehre der vorliegenden Erfindung kann ein Display beim medizinischen Fluidmanagementgerät 501 fehlen. In diesem Fall ist die Ausgabevorrichtung 503 in einer vereinfachten Form ausgeführt, beispielsweise unter Verwendung von 7- Segment LED Anzeigen oder ähnlichem. Im Einklang mit der Lehre der vorliegenden Erfindung kann eine Ausgabevorrichtung 503 aber auch vollständig fehlen. In einer alternativen Ausführungsform sind die Eingabevorrichtung 502 und die Ausgabevorrichtung 503 in einer Baugruppe, beispielsweise als Touchscreendisplay, vereint. Weiterhin verfügt das medizinische Fluidmanagementgerät 503 über eine Steuervorrichtung 504, welche zumindest eine CPU oder einen Mikrokontroller umfasst. Die Steuervorrichtung verfügt über einen internen Speicher 506, in den über die Datenschnittstelle 505 Computerprogramme geladen werden können, die die Steuervorrichtung programmieren.

Der mobile Computer 510 ist schematisch in Figur 5 dargestellt. Dieser verfügt über eine Steuervorrichtung 514, einen internen Speicher 516, der über eine Datenschnittstelle 513 mit Computerprogrammen geladen werden kann, die die Steuervorrichtung 514 programmieren, sowie eine Eingabevorrichtung 512 und eine Ausgabevorrichtung 513, die auch in einer kombinierten Ein-/Ausgabevorrichtung vereint sein können. Diese ist beispielsweise im Falle der Ausführungsbeispiele Smartphone oder Tablet-PC für den mobilen Computer 510 vorteilhaft ein Touchscreendisplay. Zusätzlich dargestellt ist in der Figur 5 schematisch ein Sensor 517 des mobilen Computers 510. Der Sensor 517 kann in schon beschriebener Weise dazu verwendet werden, Sensordaten das medizinische Fluidmanagementgerät 501 und/oder eine Behandlung, die mit dem medizinischen Fluidmanagementgerät 501 durchgeführt wird, betreffend, zu erfassen. Die Sensordaten können in der Steuervorrichtung 514 datentechnisch verarbeitet werden, oder über die Datenschnittstelle 513 und die Datenverbindung 520 an das medizinischen Fluidmanagementgerät 501 weitergegeben werden. In jedem Fall können die Sensordaten dazu verwendet werden, das medizinischen Fluidmanagementgerät 501 und/oder eine Behandlung, die mit dem medizinischen Fluidmanagementgerät 501 durchgeführt wird, zu überwachen und/oder zu steuern.

Die Datenverbindung 520 stellt eine in der Regel bidirektionale Verbindung zwischen medizinischem Fluidmanagementgerät 501 und dem mobilem Computer 510 her. Die Datenverbindung 520 kann in schon beschriebener Form auch drahtlos erfolgen. Über die Datenverbindung können Anzeigeinformation vom medizinischen Fluidmanagementgerät 501 an den mobilen Computer 510 und Eingabeinformationen und/oder Steuersignale vom mobilen Computer 510 an das medizinische Fluidmanagementgerät 501 gesendet werden.

Über die Ankoppelvorrichtung 521 kann in schon beschriebener Weise eine mechanische Ankopplung des mobilen Computers 510 an das medizinischen Fluidmanagementgerät 501 hergestellt werden.

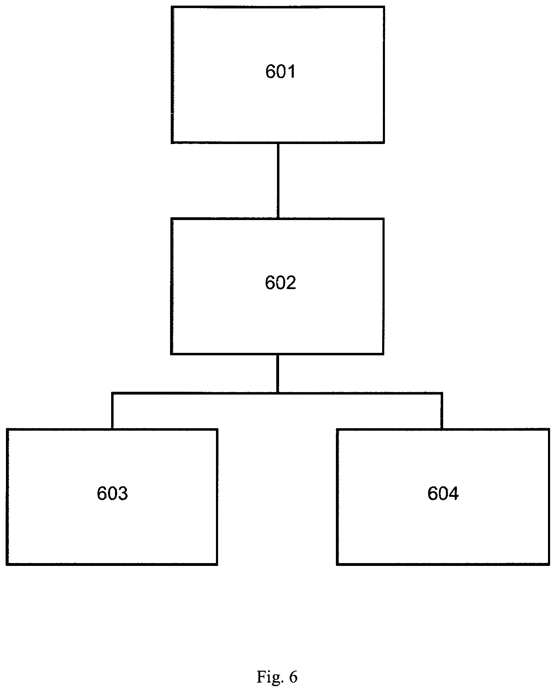

Die Figur 6 zeigt schematisch die Darstellung eines Verfahrens im Einklang mit der Lehre der vorliegenden Erfindung. Im Schritt 601 erfolgt das mechanische Ankoppeln des mobilen Computers 510 an das medizinische Fluidmanagementgerät 501. Danach erfolgt im Schritt 602 das Herstellen einer Datenverbindung zwischen medizinischem Fluidmanagementgerät 501 und mobilem Computer 510.

In den Schritten 603 erfolgt das Anzeigen von Informationen das medizinische Fluidmanagementgerät 501 oder eine Behandlung, die mit dem medizinischen Fluidmanagementgerät 501 durchgeführt wird, betreffend, über die Ausgabevorrichtung 513 des mobilen Computers. Alternativ oder ebenfalls erfolgt im Schritt 604 das Bedienen des medizinischen Fluidmanagementgeräts 501 über eine Eingabevorrichtung des mobilen Computers 510.

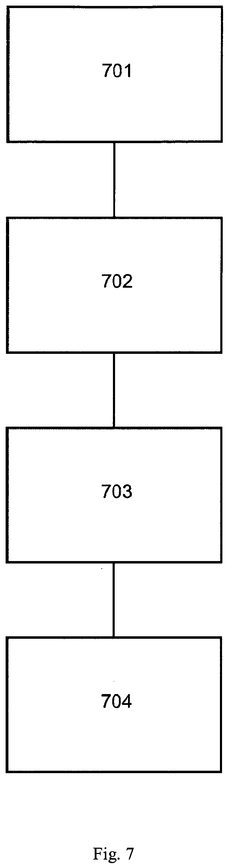

Die Figur 7 zeigt schematisch die Darstellung eines weiteren Verfahrens im Einklang mit der Lehre der vorliegenden Erfindung. Im Schritt 701 erfolgt das mechanische Ankoppeln des mobilen Computers 510 an das medizinische Fluidmanagementgerät 501 derart, dass ein Sensor des mobilen Computers 510 Sensorwerte das medizinische Fluidmanagementgerät oder eine Behandlung, die mit dem medizinischen Fluidmanagementgerät durchgeführt wird, betreffend, aufnehmen kann.

Dies erfolgt gemäß bevorzugter Ausführungsform in schon beschriebener Weise dadurch in dem die Ankopplungsvorrichtung 521 dreh- oder schwenkbar, oder ausziehbar ausgeführt ist, so, dass ein Sensor des mobilen Computers, beispielsweise eine Kamera, auf das medizinische Fluidmanagementgerät 501 oder Teile davon, beispielsweise auf eine venöse Tropfkammer, oder auf einen Patienten, der mit dem medizinische Fluidmanagementgerät 501 behandelt wird, gerichtet werden kann.

Im Schritt 702 erfolgt das Herstellen einer Datenverbindung zwischen medizinischem Fluidmanagementgerät 501 und mobilem Computer 510.

Die Schritte 701 und 702 können zeitlich auch vertauscht durchgeführt werden.

Im Schritt 703 erfolgt das Aufnehmen der Sensorwerte, und im Schritt 704 erfolgt die datentechnische Verarbeitung der Sensorwerte und davon abhängig die Überwachung und/oder Steuerung des medizinisches Fluidmanagementgeräts 501 oder einer Behandlung, die mit dem medizinischen Fluidmanagementgerät durchgeführt wird.

Die datentechnische Verarbeitung der Sensorwerte kann hierbei sowohl im mobilen Computer 510, als auch im medizinischen Fluidmanagementgerät 501 oder in beiden erfolgen. Die datentechnische Verarbeitung der Sensorwerte kann beispielsweise umfassen: die Überprüfung des Füllstands einer Flüssigkeit in einem Gefäß, die Überprüfung des Volumenstrom einer Flüssigkeit in einem Gefäß, die Überprüfung der Körpertemperatur eines Patienten, die Überprüfung der Identität des Benutzers des mobilen Computers 510, das Erkennen einer Geste eines Patienten oder die Feststellung des Standorts des mobilen Computers.

Abhängig von dem Ausgang der datentechnischen Verarbeitung kann das medizinische Fluidmanagementgerät 501 oder eine Behandlung, die mit dem medizinischen Fluidmanagementgerät 501 durchgeführt wird, in schon beschriebener Weise überwacht und/oder gesteuert werden.

Die offenbarte Lehre ermöglicht die vielseitige Erweiterung von bestimmten medizinischen Fluidmanagementgeräten um Funktionen, für die sie bislang nicht eingerichtet waren, in dem diese Funktionen mobile Computer übernehmen. Weiterhin können die Kosten und der Aufwand für die Neuerstellung von medizinischen Fluidmanagementgeräten erheblich reduziert werden, in dem auf die Ausrüstung mit teuren Ein-Ausgabevorrichtungen wie Touchscreendisplays verzichtet wird. Mobilen Computer sind im Gegensatz zu medizinischen Fluidmanagementgeräten Massenprodukte und deshalb trotz Ihrer Vielseitigkeit preiswert. Die offenbarte Lehre macht sich diese Eigenschaft zu Nutze und wendet sie vorteilhaft bei medizinischen Fluidmanagementgeräten an.

## Patentansprüche

1. Medizinisches Fluidmanagementgerät mit einer Steuervorrichtung, einer Schnittstelle, die zum Datenaustausch mit einem mobilen Computer ausgebildet ist, und mit einer mechanischen Ankoppelvorrichtung, die zum Ankoppeln an ein entsprechend ausgebildetes Gegenstück an dem mobilen Computer ausgebildet ist, und wobei die Steuervorrichtung dazu eingerichtet ist, über die Schnittstelle Informationen das medizinische Fluidmanagementgerät und/oder eine Behandlung, die mit dem medizinischen Fluidmanagementgerät durchgeführt wird, betreffend, an den mobilen Computer zu senden und/oder Bedieneingaben, die am mobilen Computer eingegeben werden, oder Steuersignale vom mobilen Computer zu empfangen
**dadurch gekennzeichnet, dass**
die mechanische Ankopplungsvorrichtung ausziehbar, dreh- oder schwenkbar ist, so dass der mobile Computer so zum medizinischen Fluidmanagementgerät ankoppelbar ist, dass vorhandene Sensoren des mobilen Computers Messwerte, die das medizinische Fluidmanagementgerät oder eine mit dem medizinischen Fluidmanagementgerät durchgeführte Behandlung betreffen, aufnehmen können.

2. Medizinisches Fluidmanagementgerät nach Anspruch 1, wobei das medizinische Fluidmanagementgerät dazu eingerichtet ist, Energie in Form elektrischer Energie an den mobilen Computer zu übertragen, wenn eine elektrische Verbindung zwischen dem medizinischen Fluidmanagementgerät und dem mobilen Computer hergestellt ist.

3. Medizinisches Fluidmanagementgerät nach einem der Ansprüche 1 oder 2, wonach das Fluidmanagementgerät ein Blutreinigungsgerät ist.

4. Blutreinigungsgerät nach Anspruch 3, ausgeführt als Dialysegerät.

5. System aus einem medizinischen Fluidmanagementgerät nach einem der Ansprüche 1 bis 4 und einem mobilen Computer, wobei der mobile Computer zur Verwendung mit dem medizinischen Fluidmanagementgerät mit einer Steuervorrichtung, einer Ein- und/oder Ausgabevorrichtung und einer Schnittstelle, die zum Datenaustausch mit einem medizinischen Fluidmanagementgerät konfiguriert ist, und mit einer mechanischen Ankoppelvorrichtung, die zum Ankoppeln an die Ankoppelvorrichtung des medizinischen Fluidmanagementgeräts ausgebildet ist, wobei die Steuervorrichtung dazu eingerichtet ist, Information das medizinischen Fluidmanagementgerät und/oder eine Behandlung, die mit dem medizinischen Fluidmanagementgerät durchgeführt wird, betreffend, auf der Ausgabevorrichtung anzuzeigen und/oder Bedienereingaben, die über die Eingabevorrichtung eingegeben werden, oder Steuersignale über die Schnittstelle an das medizinische Fluidmanagementgerät weiter zu geben,
weiterhin umfassend zumindest einen Sensor, der nach Ankoppeln des mobilen Computers an das medizinische Fluidmanagementgerät zumindest einen Messwert, der das medizinische Fluidmanagementgerät oder eine mit dem medizinischen Fluidmanagementgerät durchgeführte Behandlung betrifft erfassen kann, wobei der zumindest eine Sensor eine Kamera, ein Mikrofon, ein GPS-Sensor oder ein Temperatursensor ist, und wobei die Steuervorrichtung dazu eingerichtet ist, mit dem erfassten Messwert den Füllstand einer Flüssigkeit in einem Gefäß, den Volumenstrom einer Flüssigkeit in einem Gefäß, die Körpertemperatur eines Patienten, eine vom Patienten ausgeführte Geste, Geräusche oder Zubehörteile des medizinischen Fluidmanagementgeräts zu erkennen, die Dichtigkeit eines extrakorporalen Blutkreislaufes zu überprüfen, Therapieoptionen frei zu geben oder den Standort des mobilen Computers zu bestimmen.

6. Vorrichtung oder System nach einem der Ansprüche 1 bis 5, wonach die Steuervorrichtung des medizinischen Fluidmanagementgeräts eingerichtet ist, die Bedieneinrichtung des medizinischen Fluidmanagementgeräts nach erfolgter Ankopplung des mobilen Computers für Bedieneingaben zu sperren, wenn der mobile Computer zur Bedieneingabe das medizinische Fluidmanagementgerät betreffend eingerichtet ist.

7. Verfahren zur Überwachung und Steuerung eines medizinischen Fluidmanagementgeräts oder einer Behandlung, die mit einem medizinischen Fluidmanagementgerät durchgeführt wird, betreffend, mit den Schritten:
Ankoppeln eines mobilen Computers an eine mechanische Ankoppelvorrichtung des medizinischen Fluidmanagementgeräts, Aufnehmen der Sensorwerte,
datentechnische Verarbeitung der Sensorwerte und davon abhängig Überwachung und/oder Steuerung des medizinischen Fluidmanagementgeräts oder einer Behandlung, die mit dem medizinischen Fluidmanagementgerät durchgeführt wird
**dadurch gekennzeichnet, dass**
die mechanische Kopplungsvorrichtung ausziehbar, dreh- oder schwenkbar ist, so dass der mobile Computer so zum Dialysegerät ankoppelbar ist,
dass vorhandene Sensoren des mobilen Computers Messwerte, die das medizinische Fluidmanagementgerät oder eine mit dem medizinischen Fluidmanagementgerät durchgeführte Behandlung betreffen, aufnehmen können.

8. Verfahren nach Anspruch 7, wonach der Sensor eine Kamera, ein Mikrofon, ein Neigungssensor, ein Erschütterungssensor, ein berührempfindlicher Sensor, ein Fingerabdrucksensor, ein GPS-Sensor oder ein Temperatursensor ist.

9. Verfahren nach einem der Ansprüche 7 oder 8, wonach das Sensorsignal dazu verwendet wird, den Füllstand einer Flüssigkeit in einem Gefäß, den Volumenstrom einer Flüssigkeit in einem Gefäß, die Körpertemperatur eines Patienten, die Identität des Benutzers zu bestimmen, eine vom Patienten ausgeführte Geste, Geräusche oder Zubehörteile des medizinischen Fluidmanagementgeräts zu erkennen, die Dichtigkeit eines extrakorporalen Blutkreislaufes zu überprüfen, Therapieoptionen frei zu geben oder den Standort des mobilen Computers zu bestimmen.

10. Verfahren nach einem der Ansprüche 7 bis 9, wonach eine Bedieneinrichtung des medizinischen Fluidmanagementgeräts nach erfolgter Ankopplung des mobilen Computers für Bedieneingaben gesperrt wird, wenn der mobile Computer zur Bedieneingabe das medizinische Fluidmanagementgerät betreffend, eingerichtet ist.

11. Verfahren nach einem der Ansprüche 7 bis 10, wonach das Fluidmanagementgerät ein Blutreinigungsgerät ist.

12. Verfahren nach Anspruch 11, wonach das Blutreinigungsgerät ein Dialysegerät ist.

## Claims

1. Medical fluid management device comprising a control apparatus, comprising an interface designed for data exchange with a mobile computer and comprising a mechanical coupling apparatus designed for coupling to a correspondingly designed counterpart on the mobile computer, wherein the control apparatus is configured to transmit, via the interface, information relating to the medical fluid management device and/or a treatment performed with the medical fluid management device to the mobile computer and/or to receive, via the interface, operator inputs entered on the mobile computer or control signals from the mobile computer,
**characterized in that**
the mechanical coupling apparatus is extendable, rotatable or pivotable such that the mobile computer is able to be coupled to the medical fluid management device in such a way that available sensors of the mobile computer can record measured values relating to the medical fluid management device or a treatment performed with the medical fluid management device.

2. Medical fluid management device according to Claim 1, wherein the medical fluid management device is configured to transfer energy in the form of electrical energy to the mobile computer when an electrical connection is established between the medical fluid management device and the mobile computer.

3. Medical fluid management device according to either of Claims 1 and 2, according to which the fluid management device is a blood-cleansing device.

4. Blood-cleansing device according to Claim 3, designed as a dialysis device.

5. System made up of a medical fluid management device according to any one of Claims 1 to 4 and a mobile computer, wherein the mobile computer is designed for use with the medical fluid management device with a control apparatus, an input and/or output apparatus and an interface configured for data exchange with a medical fluid management device, and with a mechanical coupling apparatus designed for coupling to the coupling apparatus of the medical fluid management device, wherein the control apparatus is configured to indicate, on the output apparatus, information relating to the medical fluid management device and/or a treatment performed with the medical fluid management device and/or to pass on operator inputs entered by way of the input apparatus or control signals to the medical fluid management device via the interface,
furthermore comprising at least one sensor which, once the mobile computer has been coupled to the medical fluid management device, can capture at least one measured value relating to the medical fluid management device or a treatment performed with the medical fluid management device, wherein the at least one sensor is a camera, a microphone, a GPS sensor or a temperature sensor and wherein the control apparatus is configured to use the captured measured value to detect the filling level of a liquid in a vessel, the volumetric flow rate of a liquid in a vessel, the body temperature of a patient, a gesture performed by the patient, noises or accessories of the medical fluid management device, to check the tightness of an extracorporeal blood circuit, to enable therapy options or to determine the location of the mobile computer.

6. Apparatus or system according to any one of Claims 1 to 5, according to which the control apparatus of the medical fluid management device is configured to block the operator appliance of the medical fluid management device for operator inputs following the coupling of the mobile computer if the mobile computer is configured for operator inputs relating to the medical fluid management device.

7. Method for monitoring and controlling a medical fluid management device or a treatment performed with a medical fluid management device, including the steps of:
coupling a mobile computer to a mechanical coupling apparatus of the medical fluid management device,
recording the sensor values,
data processing the sensor values and, depending thereon, monitoring and/or controlling the medical fluid management device or a treatment performed with the medical fluid management device,
**characterized in that**
the mechanical coupling apparatus is extendable, rotatable or pivotable such that the mobile computer is able to be coupled to the dialysis device in such a way that available sensors of the mobile computer can record measured values relating to the medical fluid management device or a treatment performed with the medical fluid management device.

8. Method according to Claim 7, according to which the sensor is a camera, a microphone, an inclinometer, a vibration sensor, a touch-sensitive sensor, a fingerprint sensor, a GPS sensor or a temperature sensor.

9. Method according to either of Claims 7 and 8, according to which the sensor signal is used to determine the filling level of a liquid in a vessel, the volumetric flow rate of a liquid in a vessel, the body temperature of a patient, the identity of the user, to detect a gesture performed by the patient, noises or accessories of the medical fluid management device, to check the tightness of an extracorporeal blood circuit, to enable therapy options or to determine the location of the mobile computer.

10. Method according to any one of Claims 7 to 9, according to which an operator appliance of the medical fluid management device is blocked for operator inputs following the coupling of the mobile computer if the mobile computer is configured for operator inputs relating to the medical fluid management device.

11. Method according to any one of Claims 7 to 10, according to which the fluid management device is a blood-cleansing device.

12. Method according to Claim 11, according to which the blood-cleansing device is a dialysis device.

## Revendications

1. Appareil de gestion de fluides médical comprenant un dispositif de commande, une interface qui est réalisée pour échanger des données avec un ordinateur mobile, et un dispositif de couplage mécanique qui est réalisé pour être couplé à une pièce conjuguée réalisée de manière correspondante sur l'ordinateur mobile, dans lequel le dispositif de commande est adapté, par l'intermédiaire de l'interface, pour envoyer à l'ordinateur mobile des informations concernant l'appareil de gestion de fluides médical et/ou un traitement qui est effectué à l'aide de l'appareil de gestion de fluides médical et/ou pour recevoir de l'ordinateur mobile des entrées d'opérateur qui sont saisies sur l'ordinateur mobile ou des signaux de commande,
**caractérisé en ce que**
le dispositif de couplage mécanique est extensible, rotatif ou pivotant, de manière à ce que l'ordinateur mobile puisse être couplé à l'appareil de gestion de fluides médical, de telle sorte que des capteurs présents de l'ordinateur mobile puissent enregistrer des valeurs de mesure qui concernent l'appareil de gestion de fluides médical ou un traitement effectué à l'aide de l'appareil de gestion de fluides médical.

2. Appareil de gestion de fluides médical selon la revendication 1, l'appareil de gestion de fluides médical étant adapté pour transmettre de l'énergie sous forme d'énergie électrique à l'ordinateur mobile lorsqu'une liaison électrique est établie entre l'appareil de gestion de fluides médical et l'ordinateur mobile.

3. Appareil de gestion de fluides médical selon l'une quelconque des revendications 1 et 2, l'appareil de gestion de fluides étant un appareil de purification du sang.

4. Appareil de purification du sang selon la revendication 3, réalisé sous forme d'appareil de dialyse.

5. Système constitué d'un appareil de gestion de fluides médical selon l'une des revendications 1 à 4, et d'un ordinateur mobile, dans lequel l'ordinateur mobile est réalisée pour l'utilisation avec l'appareil de gestion de fluides médical comprenant un dispositif de commande, un dispositif d'entrée et/ou de sortie et une interface configurée pour échanger des données avec un appareil de gestion de fluides médical, et comprenant un dispositif de couplage mécanique qui est réalisé pour être couplé au dispositif de couplage de l'appareil de gestion de fluides médical, dans lequel le dispositif de commande est adapté pour afficher sur le dispositif de sortie des informations concernant l'appareil de gestion de fluides médical et/ou un traitement qui est effectué à l'aide de l'appareil de gestion de fluides médical et/ou pour retransmettre à l'appareil de gestion de fluides médical des entrées d'opérateur, qui sont saisies par l'intermédiaire du dispositif d'entrée, ou des signaux de commande par le biais de l'interface, comprenant en outre au moins un capteur qui, après que l'ordinateur mobile a été couplé à l'appareil de gestion de fluides médical, peut détecter au moins une valeur de mesure qui concerne l'appareil de gestion de fluides médical ou un traitement effectué à l'aide de l'appareil de gestion de fluides médical, dans lequel ledit au moins un capteur est une caméra, un microphone, un capteur GPS ou un capteur de température, et dans lequel le dispositif de commande est adapté pour reconnaître, à l'aide de la valeur de mesure détectée, le niveau d'un liquide dans un récipient, le débit volumique d'un liquide dans un récipient, la température corporelle d'un patient, un geste effectué par le patient, des sons ou des accessoires de l'appareil de gestion de fluides médical, pour vérifier l'étanchéité d'un circuit sanguin extracorporel, pour valider des options thérapeutiques ou pour déterminer l'emplacement de l'ordinateur mobile.

6. Dispositif ou système selon l'une quelconque des revendications 1 à 5, le dispositif de commande de l'appareil de gestion de fluides médical étant adapté pour bloquer le dispositif d'opérateur de l'appareil de gestion de fluides médical vis-à-vis d'entrées d'opérateur après que l'ordinateur mobile a été couplé avec succès, lorsque l'ordinateur mobile est adapté pour une entrée d'opérateur qui concerne l'appareil de gestion de fluides médical.

7. Procédé de surveillance et de commande d'un appareil de gestion de fluides médical ou d'un traitement qui est effectué à l'aide d'un appareil de gestion de fluides médical, comprenant les étapes suivantes :
couplage d'un ordinateur mobile à un dispositif de couplage mécanique de l'appareil de gestion de fluides médical,
enregistrement des valeurs de capteurs,
traitement technique de données des valeurs de capteurs et, en fonction de celles-ci, surveillance et/ou commande de l'appareil de gestion de fluides médical ou d'un traitement qui est effectué à l'aide de l'appareil de gestion de fluides médical,
**caractérisé en ce que**
le dispositif de couplage mécanique est extensible, rotatif ou pivotant, de manière à ce que l'ordinateur mobile puisse être couplé à l'appareil de dialyse,
de telle sorte que des capteurs présents de l'ordinateur mobile puissent enregistrer des valeurs de mesure qui concernent l'appareil de gestion de fluides médical ou un traitement effectué à l'aide de l'appareil de gestion de fluides médical.

8. Procédé selon la revendication 7, selon lequel le capteur est une caméra, un microphone, un capteur d'inclinaison, un capteur de vibrations, un capteur tactile, un capteur d'empreintes digitales, un capteur GPS ou un capteur de température.

9. Procédé selon l'une des revendications 7 et 8, selon lequel le signal de capteur est utilisé pour déterminer le niveau d'un liquide dans un récipient, le débit volumique d'un liquide dans un récipient, la température corporelle d'un patient, l'identité de l'utilisateur, pour reconnaître un geste effectué par le patient, des sons ou des accessoires de l'appareil de gestion de fluides médical, pour vérifier l'étanchéité d'un circuit sanguin extracorporel, pour valider des options thérapeutiques ou pour déterminer l'emplacement de l'ordinateur mobile.

10. Procédé selon l'une des revendications 7 à 9, selon lequel un dispositif d'opérateur de l'appareil de gestion de fluides médical est bloqué vis-à-vis d'entrées d'opérateur après que l'ordinateur mobile a été couplé avec succès, lorsque l'ordinateur mobile est adapté pour une entrée d'opérateur qui concerne l'appareil de gestion de fluides médical.

11. Procédé selon l'une des revendications 7 à 10, selon lequel l'appareil de gestion de fluides est un appareil de purification du sang.

12. Procédé selon la revendication 11, selon lequel l'appareil de purification du sang est un appareil de dialyse.
